# EUROPEAN PATENT SPECIFICATION

(11) **EP 1 556 139 B1**
(45) Date of publication and mention of the grant of the patent: **19.07.2006**
(21) Application number: 03773833.3
(22) Date of filing: 27.10.2003
(51) Int. Cl.: A61N 5/06

(54) **TANNING APPARATUS AND METHOD THEREOF**
BRÄUNUNGSAPPARAT UND ZUGEHÖRIGES VERFAHREN
APPAREIL DE BRONZAGE ET PROCEDE ASSOCIE

(30) Priority: 29.10.2002 GB 0225132
(43) Date of publication of application: 27.07.2005
(73) Proprietor: Andrew, Carr, Fairweather Green, Bradford BD8 0EZ (GB)
(72) Inventor: Andrew, Carr, Fairweather Green, Bradford BD8 0EZ (GB)
(74) Representative: Harrison Goddard Foote
(86) International application number: PCT/GB2003/004626
(87) International publication number: WO 2004/039452

(56) References cited:
- DE-A- 3 035 624
- DE-A- 19 502 980
- DE-A- 19 753 277
- US-A- 4 839 513
- US-A- 5 466 248
- US-A1- 2003 060 853

## Description

### Field of Invention

This invention relates to a tanning unit for legs. The tanning unit artificially tans a person's legs by emitting tanning radiation. Such a tanning unit may be used with a tanning chamber. The invention also relates to a tanning chamber provided with such a tanning unit.

### Background to the Invention

Artificial sun tanning systems that irradiate the skin are very popular, especially in winter months when a natural tan can not be readily acquired or in regions where the summer tanning season is relatively short.

There are essentially two types of tanning chamber; a horizontal tanning chamber and a vertical tanning chamber. A horizontal tanning chamber enables a person to obtain an artificial sun tan while lying down. A vertical tanning chamber enables a person to obtain an artificial sun tan while standing up.

Conventional horizontal tanning chambers include an upper tanning portion and a lower tanning portion. Tanning lamps are arranged on the inner walls of both the upper and lower portions. The upper tanning portion and lower tanning portion are connected by hinges so that the chamber can be opened and closed in a manner analogous to a shell. When a user lies down on the lower tanning portion and the chamber is closed they are generally surrounded by tanning lamps and can be irradiated from almost every direction.

Conventional vertical tanning chambers are generally upright tubular structures. Tanning lamps are arranged on the inner wall of the chamber such that when a user stands inside they are generally surrounded by lamps and can be irradiated from almost every direction.

Neither the horizontal tanning chamber nor the vertical tanning chamber provide a uniform sun tan.

It has been found that, due to the size and shape of a user's body, the upper body tans much more quickly and easily than the lower body. The lower body region, e.g. the legs, is much narrower in shape than the upper body and so more difficult to irradiate. When a user lies in the centre of the horizontal tanning chamber or stands in the centre of a vertical tanning chamber the lower body is further away from the tanning lamps.

As mentioned above, a user of a horizontal tanning chamber lies over the tanning lamps arranged within the lower tanning portion. It has been found that the surface of the user facing the tanning lamps in the lower tanning portion of a horizontal tanning chamber tans much more quickly and easily than the surface of the user facing the tanning lamps in the upper tanning portion. A certain amount of overhead space is required in a horizontal tanning chamber, and therefore the lamps in the upper tanning portion are positioned further away from the person than the lamps in the lower tanning portion.

Furthermore, it has been found that the surface of the outer part of a user's leg tans more quickly and easily than the surface of the inner part of the legs. When a person lies or stands in a natural and comfortable position within a conventional tanning chamber the inside surface of each leg is irradiated much less than the outside surface of each leg. It is unnatural and uncomfortable for a person to lie with their legs ajar so that they are facing the tanning lamps in the upper tanning portion of a horizontal tanning chamber. A person can not be expected to hold this position for any meaningful length of time. It is also unsafe and unnatural for a person to stand with their legs twisted in an outward position so that they are facing the tanning lamps in a vertical tanning chamber. The position is unstable and the user is at risk of falling over. Ironically, even if the legs of the user are held ajar and twisted so that the inner surface of the legs can be irradiated the whole of each leg would still not be uniformly sun tanned. When the legs are turned so that the inner surfaces can face the tanning lamps, the outer surface of each leg now faces away from the tanning lamps and so receives far less irradiation.

Attempts have been made to try and overcome the problem of uneven tanning by adding extra lamps to the lower half of the vertical and horizontal chambers. Documents DE 195 02 980 and DE 197 53 277 disclose such chambers. However, while this solution helps to provide a more even tan between the upper body and the outside portion of the lower body and also the surface facing the tanning lamps of the upper portion and the surface facing the tanning lamps of the lower portion, the variation in sun tan between the outer and inner parts of the legs of the user is exacerbated.

A separate leg-tanning machine has also been developed to help 'top-up' a sun tan to the legs. In this case, a person must lie down and then place their legs within the machine. The machine has two sections, one for each leg. Tanning tubes are arranged within each section of the machine so that they surround the entire leg and both the inner and outer surface of the legs are irradiated. The use of this machine is supplemental to that of the tanning chamber. Thus it would be inconvenient, time consuming and expensive for a person to use both machines in order to achieve a more uniform sun tan. Such a leg-tunning apparatus is known from US 2003/060853 which was published after the priority date of the Application.

It is the aim of the embodiments of the present invention to mitigate at least some of the problems mentioned above.

### Statement of Invention

According to a first aspect of the present invention there is provided a tanning unit for legs comprising a housing with at least one outer surface and at least one tanning lamp wherein the at least one lamp is arranged within the housing and disposed such that, during operation, tanning radiation is emitted through the at least one outer surface, and wherein the housing is shaped to enable a user to straddle the tanning unit such that the inside surface of each leg of the user is adjacent the at least one outer surface, whereby, in operation, the inside surfaces of both legs are simultaneously subject to said tunning radiation.

Preferably, the at least one tanning lamp is arranged within the housing and disposed adjacent the outer surface.

Conveniently, the housing has at least two outer surfaces. Advantageously, the at least one tanning lamp is arranged within the housing and disposed adjacent each one of the at least two outer surfaces.

Preferably, the tanning unit further comprises control means to control the operation of the at least one tanning lamp.

Conveniently, the tanning unit further comprises at least one shield mounted over the at least one outer surface and formed from material transmissive to tanning radiation.

According to a second aspect of the invention there is provided a tanning chamber that includes a tanning unit for legs which comprises a housing with at least one outer surface and at least one tanning lamp wherein the at least one lamp is arranged within the housing and disposed such that, during operation, tanning radiation is emitted through the at least one outer surface, and wherein the housing is shaped to enable a user to straddle the tanning unit such that the inside surface of each leg of the user is adjacent the at least one outer surface.

Preferably, the tanning unit for legs is integrally fitted within the chamber.

Conveniently, the tanning unit for legs is operable independently of the chamber. Alternatively, the tanning unit for legs is operable together with the chamber.

Advantageously, the tanning chamber is a vertical tanning chamber to tan a user while in a standing position. Even more preferably, the tanning chamber further comprises a housing with a floor section wherein the tanning unit for legs is located on the floor section such that a user may straddle the unit while standing.

Alternatively, the tanning chamber is a horizontal tanning chamber to tan a user while in a lying position. The horizontal tanning chamber may comprise an upper tanning portion and a lower tanning portion, wherein the tanning unit for legs is fitted at one end of the lower tanning portion such that a user may straddle the unit while lying down.

According to a third aspect of the invention there is provided a method of tanning legs using a tanning unit for legs as claimed in any of claims 1 to 6 comprising the steps of:
a user straddling the tanning unit such that the inside surface of each leg of the user is adjacent the at least one outer surface of the tanning unit; and
irradiating the inside surface of each leg with tanning radiation when the tanning unit is in operation.

According to a fourth aspect of the invention there is provide a method of tanning using a vertical tanning chamber including a tanning unit for legs as claimed in any of claims 11 or 12, when dependent on any of claims 1 to 10, comprising the steps of:
a user standing within the vertical tanning chamber and straddling the tanning unit for legs whereby the user is generally surrounded by tanning lamps of the tanning chamber and the inside surface of each leg of the user is adjacent the at least one outer surface of the tanning unit; and
irradiating, via the tanning lamps of the vertical tanning chamber, the entire length of the user's body from a plurality of directions and irradiating, via the tanning unit for legs, the inside surface of each leg.

According to a fifth aspect of the invention there is provided a method of tanning using a horizontal tanning chamber including a tanning unit for legs as claimed in claims 13 or 14, when dependent on any of claims 1 to 10, comprising the steps of
a user lying within the horizontal tanning chamber and straddling the tanning unit for legs whereby the user is generally surrounded by tanning lamps of the tanning chamber and the inside surface of each leg of the user is adjacent the at least one outer surface of the tanning unit; and
irradiating, via the said tanning lamps of the tanning chamber, the entire length of the user's body from a plurality of directions and irradiating, via the tanning unit, the inside surface of each leg.

Embodiments of the present invention overcome the problem of sun tanning the inside surface of legs. Embodiments of the present invention provide a tanning unit for legs to suntan the inside surface of each leg of the user.

Embodiments of the present invention provide a tanning unit for legs which is straddled by the user so that the inside surface of each leg of the user is adjacent a surface through which tanning radiation is emitted.

Embodiments of the present invention overcome the problem of an uneven suntan between the inside and outside surfaces of the legs of the user. Embodiments of the present invention provide a tanning chamber that includes a tanning unit for legs to uniformly irradiate the inside and outside surfaces of the legs of the user with tanning radiation.

Embodiments of the present invention overcome the problem of an uneven suntan between the upper body and lower body of the user. Embodiments of the present invention provide a tanning chamber that includes a tanning unit for legs to uniformly irradiate the whole body of a user with tanning radiation.

### Brief Description of the Drawings

Embodiments of the present invention are illustrated in the accompanying drawings, by way of example only, in which:
Figure 1 depicts a perspective view of a conventional horizontal tanning chamber;
Figure 2 depicts a perspective view of a conventional vertical tanning chamber;
Figure 3 depicts a perspective view of an embodiment of a tanning unit;
Figure 4 depicts a perspective view of a further embodiment of a tanning unit
Figure 5A depicts a view illustrating a first position of a user straddling a tanning unit;
Figure 5B depicts a view illustrating a second position of a user straddling a tanning unit;
Figure 6 depicts a perspective view of a horizontal tanning chamber including a tanning unit;
Figure 7 depicts a perspective view of a vertical tanning chamber including a tanning unit.

### Detailed Description of the Invention

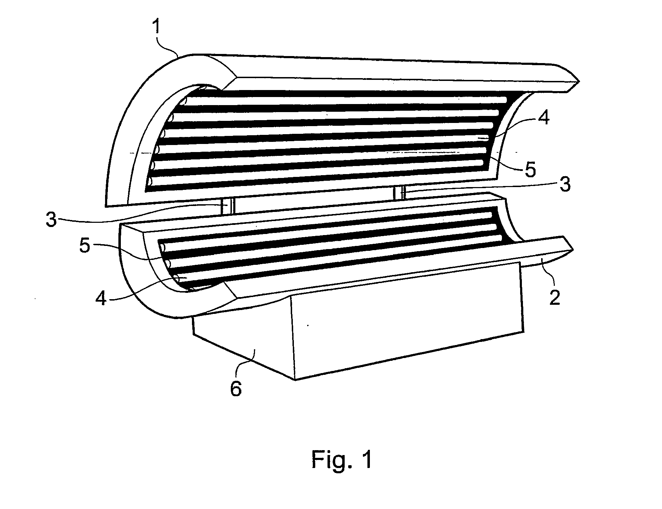

Figure 1 depicts a conventional horizontal tanning chamber. The horizontal tanning chamber includes an upper tanning portion (1) and a lower tanning portion (2). The upper tanning portion (1) and lower tanning portion (2) are connected by hinges (3) so that the upper tanning portion (1) can be moved upwards and downwards to open and close the chamber in a manner analogous to a shell. The lower tanning portion (2) is supported by a base (6). Tanning lamps (4) are arranged on the inner walls (5) of the upper and lower portions (1,2). When the chamber is closed a void is formed that is essentially tubular is shape. When the horizontal tanning chamber is in use, a person lies within the void on the lower tanning portion (2). A person skilled in the art will understand that the user is generally surrounded by tanning lamps (4) and irradiated from almost every direction.

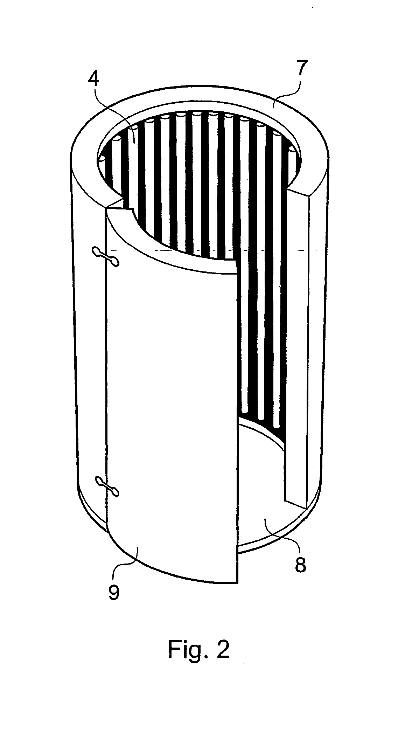

Figure 2 depicts a conventional vertical tanning chamber. The vertical tanning chamber includes a vertical wall (7) and floor (8). At least one door (9) is formed within the wall (7) so that a user can enter and exit the chamber. Tanning lamps (4) are arranged on the inner surface of the wall (7) and door (9). The internal configuration of the chamber is generally circular in shape. When the door (9) is closed a void is formed that is essentially tubular in shape. When the vertical tanning chamber is in use, a person stands on the floor (8) within the void. A person skilled in the art will understand that the user is generally surrounded by lamps (4) and the entire length of the body is irradiated from almost every direction.

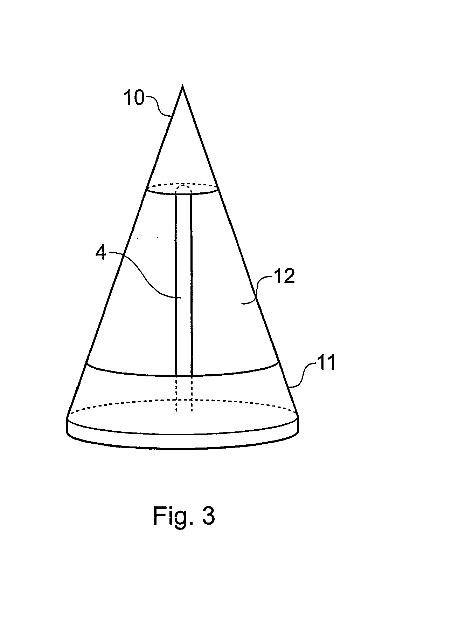

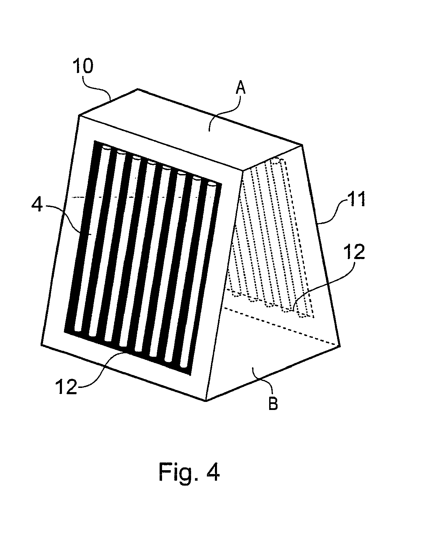

Figure 3 depicts an embodiment of the tanning unit for legs (10) according to the present invention. The tanning unit (10) is shaped like a cone. The tanning unit comprises a housing (11) with an outer surface (12) that is transmissive to tanning radiation and one tanning lamp (4). Figure 4 depicts a further embodiment of the tanning unit for legs (10) according to the present invention. This particular embodiment of the tanning unit (10) is shaped like a four-sided pyramid with a flat top. The tanning unit (10) comprises a housing (11) with two outer surfaces (12) that are transmissive to tanning radiation and a multitude of tanning lamps (4). The outer surfaces (12) are arranged on opposing sides of the unit (10).

The tanning unit (10) of the present invention has at least one outer surface and at least one tanning lamp (4). However, a person skilled in the art will understand that the tanning unit (10) can have any number of outer surfaces (12) and tanning lamps (4).

The outer surfaces (12) of the tanning unit for legs (10) may be formed by a material that is transmissive to tanning radiation. Alternatively, the outer surfaces (12) may be holes formed in the housing (11) of the unit (10). The rest of the housing may be formed from a material that does not transmit tanning radiation, such as extruded aluminium, aluminium alloy or plastic. In Figure 4, the top (A) and base (B) are formed from material that is opaque to tanning radiation.

The tanning lamps (4) of the present invention are arranged within the housing (11). They are disposed relative to the outer surfaces (12) such that tanning radiation is emitted through them when the unit (10) is in operation. The tanning unit (10) may include a single tanning lamp (4) arranged within the centre of the housing (12) as depicted in Figure 3. Alternatively, the tanning unit (10) may include a plurality of tanning lamps (4) arranged within the centre of the housing (11).

The tanning unit (10) may instead include at least one lamp (4) disposed adjacent each of the outer surfaces (12). Figure 4 depicts a tanning unit (10) with a plurality of tanning lamps (4) disposed adjacent each one of the two outer surfaces (12).

The tanning lamps (4) emit radiation within a limited spectral range of the ultraviolet region know as UVA and UVB. UVA and UVB light have a wavelength range of 280 to 400 nanometers. The tanning lamps (4) may be elongate tubes such as low-pressure fluorescent tubes. Alternatively, the tanning lamps (4) may be high-pressure bulbs. The tanning unit (10) may include up to approximately fifty tanning lamps (4). The wattage of the lamps (4) may vary from approximately 5W to approximately 1000W.

The housing (11) is shaped and sized such that it has a width and depth sufficient to accommodate the number of tanning lamps (4) required to provide a sun tan within a reasonable period of time and also enable the tanning lamps (4) to be arranged within the housing (11) as required.

The tanning lamps (4) may be held in position within the housing (11) using lamp holders (not shown). The tanning lamps (4) may be spaced at least a 'finger width apart' in order to facilitate installation and removal. The lamp holders may be located within the housing so that the ends of the tanning lamps (4) may be flush. Consequently, the lamps (4) are arranged in a uniform row. Alternatively, only the end of every other tanning lamp (4) may be flush and the lamps (4) arranged in a staggered configuration. The tanning lamps (4) may be staggered to ensure tanning radiation irradiates the entire length of the user's legs. The tanning lamps (4) may be staggered to maximise the number of lamps (4) within the housing (11). Or the tanning lamps (4) may be staggered to minimise the size of the housing (11). Figure 4 depicts a tanning unit (10) with a uniform row of tanning lamps (4) disposed adjacent each outer surface (12).

The housing (11) of the tanning unit (10) is shaped such that a user is able to straddle the unit (10) when standing up or lying down. When a user straddles the tanning unit (10) the inside surface of each leg faces an outer surface (13). Thus, when the tanning unit (10) is in operation, the inside surface of each leg is irradiated by tanning radiation.

A tanning unit (10) suitable for straddling in the standing position may have a height of approximately 20cm to approximately 120cm. If the tanning unit (10) is conical or dome-like, the base may have a diameter of approximately 4cm to approximately 50cm. Alternatively, if the tanning unit (10) is four-sided pyramid, rectangular or square-like box the width may be approximately 4cm to approximately 50cm and the depth may be approximately 4cm to approximately 60cm. The tanning unit (10) may be shaped such that it tapers upwards in order to accommodate the V-shape of the user's straddling legs.

A tanning unit (10) suitable for straddling while lying down may have length of approximately 20cm to approximately 120cm. If the tanning unit (10) is conical or dome-like the maximum diameter may be approximately 4cm to approximately 50cm. If the tanning unit (10) is a four-sided pyramid, rectangular or square box then the width may be approximately 4cm to approximately 50cm and the height may be approximately 4cm to approximately 40cm. The tanning unit (10) may taper at one end in order to accommodate the V-shape of the user's straddling legs.

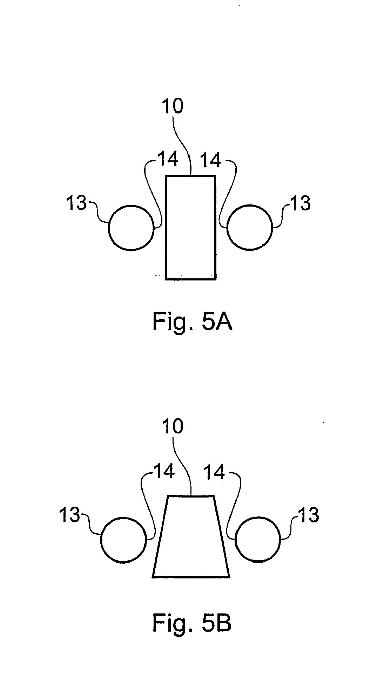

Figures 3 and 4 depict a tanning unit that is suitable for a user to straddle in the standing position. Figure 3 depicts a conical tanning unit (10) that tapers upwardly in order to accommodate the V-shape of the user's straddling legs. Figure 4 depicts a tanning unit (10) that tapers upward in a manner similar to a pyramid such that it is much wider at the bottom than at the top. Figure 5A illustrates a user straddling a tanning unit (10) while standing up. Figure 5B illustrates a user straddling a tanning unit (10) while lying down. Both figures 5A and 5B show that when a user straddles a tanning unit (10) the inside surface (14) of each leg (13) is adjacent an outer surface (12).

The tanning lamps (4) may be arranged within the housing (11) such that they extend in a direction that is generally parallel to the legs of the user (14). When the user straddles the housing (11) in a standing position the lamps (4) may be arranged vertically within the housing (11) so that they are generally parallel to the legs of the user (13). When the user straddles the housing (11) in a lying down position the tanning lamps (4) may be arranged horizontally so that they are generally parallel to the legs of the user (13). Alternatively, the tanning lamps (4) are arranged within the housing (11) such that they extend in a direction that is generally perpendicular to the legs of the user (11). Thus, the lamps (4) may be arranged horizontally in the housing (11) when a user straddles the unit in a standing position or arranged vertically in the housing when a user straddles the unit in a lying down position. Figures 3 and 4 depict a tanning unit (10) with tanning lamps (4) arranged vertically within the housing. When a user straddles the tanning unit (10) of figures 3 or 4 in a standing position the tanning lamps (4) extend in a direction that is generally parallel to the legs of the user (13).

The tanning unit (10) includes control means (not shown) to control the operation of the tanning lamps (4). The control means include ballast or electronic chokes, capacitors, starters, switches and timers. The control means may be accommodated within the housing (11) of the unit (10). Certain control means, such as timers, on/off switches, emergency switches etc. may be located on the outside of the housing (11) so that they are easily accessible to the user. The tanning lamps (4) may be electrically connected to the control means via the lamp holders (not shown). The control means may provide control of each separate or groups of tanning lamps (4). The control means may provide control of the tanning lamps (4) disposed relative to certain outer surfaces (12). The tanning unit (10) may be powered by an external power supply or an internally fitted battery (not shown).

The tanning unit (10) may also include at least one shield (not shown) to protect the user from direct contact with the tanning lamps (4). The shields are mounted over each of the outer surfaces (12). The shields may be moulded to fit the housing (11) and also curved to match the shape of the user's legs. The shields may be concave to allow the legs of the user to fit neatly next to the outer surfaces (12). The shields are formed from material that is transmissive to tanning radiation, such as acrylic. The shields may be a sheet, grill or mesh. Supports may be used to structurally reinforce the shields. The supports may be formed from aluminium or aluminium alloy.

The tanning unit (10) may include cooling means (not shown) to dissipate the heat created by the tanning lamps (4). The cooling means may include cooling fins and/or a fan.

Reflecting means (not shown) may be arranged within the housing (11) to reflect stray tanning radiation towards the outer surfaces (12). The reflective surfaces may be located to the side and/or behind the tanning lamps (4). The reflective surfaces may be formed from a material that reflects tanning radiation such as flexible polished sheet metal or metallised plastics.

The tanning unit for legs (10) may be a portable stand-alone unit. The tanning unit (10) may be shaped and sized so that it can be lifted and carried by a person to a convenient location for use. The tanning unit (10) may be used as a stand-alone unit separate from a tanning chamber.

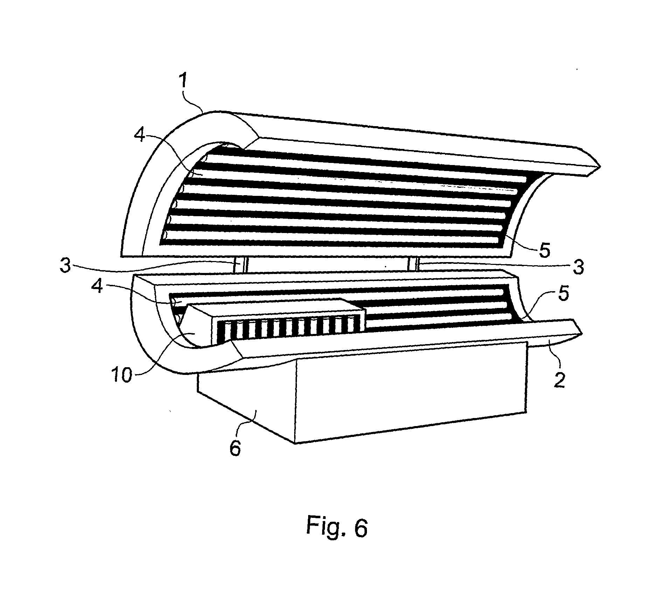

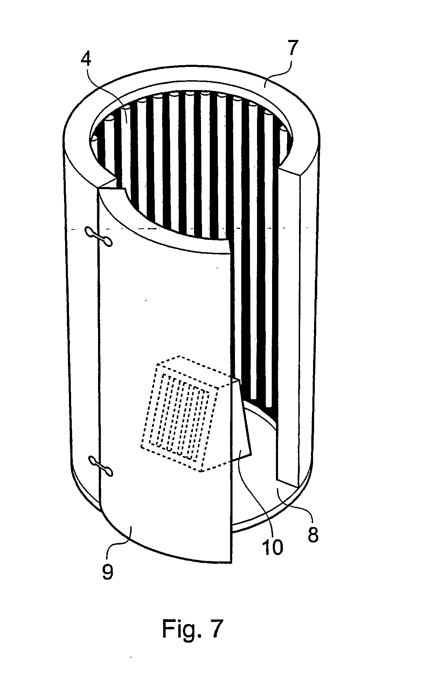

In a further aspect of the invention, the tanning unit for legs (10) may be included in a tanning chamber. Figure 6 depicts a horizontal tanning chamber including a tanning unit (10). Figure 7 depicts a vertical tanning chamber including a tanning unit (10)

The tanning chamber of figure 6 includes an upper tanning portion (1) and lower tanning portion (2). The upper tanning portion (1) and lower tanning portion (2) are connected by hinges (3) so that the upper tanning portion (1) can be moved upwards and downwards to open and close the chamber in a manner analogous to a shell. The lower tanning portion is supported by a base (6). Tanning lamps (4) are arranged on the inner walls (5) of the upper and lower portions (1,2). A tanning unit (10) is arranged on the inner wall (5) of the lower tanning portion (2). The tanning unit (10) is located at one end of the tanning chamber so that the legs of the user can straddle the unit when lying down on the lower tanning portion (2).

The tanning unit (10) may be retrofitted within the tanning chamber, so that it can be added or removed as required. Alternatively, the tanning unit (10) may be integrally fitted within the tanning chamber.

The horizontal tanning chamber may be approximately 2040mm long and approximately 1230mm wide. The tanning chamber may have a height of approximately 1660mm when open and approximately 1160mm when closed.

The tanning lamps (4) of the horizontal tanning chamber emit UVA and UVB radiation. The tanning lamps (4) may be elongate tubes such as low-pressure fluorescent tubes. The horizontal tanning chamber may include high output tubes that range from approximately 80W to approximately 200W and/or booster tubes that range from approximately 15W to approximately 36W. The tanning chamber may include approximately 30 to approximately 90 tanning lamps (4). The lamps (4) are often aligned horizontally, parallel with the plane of the floor, and may extend the full length of the user. Booster tubes may be added towards ends of the upper and/or lower tanning portions (1,2) to boost the tanning of the face and/or the outer surface of the user's legs. Booster tubes may also be added to the middle section of the upper and/or lower portions (1,2) to boost the tanning of the body.

The tanning lamps (4) of the horizontal tanning chamber may be held in place by lamp holders (not shown). The ends of the tanning lamps (4) may be flush so that the lamps (4) are arranged in a uniform row. Alternatively, the end of every other tanning lamp (4) may be flush so that the lamps (4) are arranged in a staggered configuration. Figure 6 shows the tanning lamps (4) arranged in horizontal uniform rows on the upper and lower portions (1,2).

The tanning lamps (4) of the chamber may be arranged at least a 'finger width' apart to facilitate installation and removal of the lamps (4).

When the horizontal tanning chamber is closed the upper and lower portion (2) form a void that is essentially tubular is shape. When the horizontal tanning chamber and tanning unit are in use, a person lies within the void on the lower tanning portion (2) and straddles the tanning unit (10). The tanning lamps (4) of the tanning chamber are arranged so that the user is generally surrounded by lamps (4) such that the entire length of the body is irradiated from almost every direction. The inside surface of each leg faces the outer surfaces (12) of the tanning unit (10) and the outside surface of each leg faces the tanning lamps (4) arranged on the inner walls (5) of the upper and lower portions (1,2). Since radiation is able to reach all the parts of the user's legs they are sun-tanned more evenly. When a user straddles the tanning unit (10) in a lying down position the outside surface of each leg is located closer to the tanning lamps (4) of the upper and lower portions (1,2) than in a conventional horizontal tanning chamber. Consequently, since the lower body of the user is located closer to the tanning lamps (4) in the horizontal tanning chamber, the overall suntan across the entire body is more uniform.

Shields (not shown) may be mounted on the inner walls the upper and lower portions (1,2) of the horizontal tanning chamber. The shields are mounted over the tanning lamps to protect the user from direct contact with the tanning lamps (4). The shields are formed from a material that is transmissive to tanning radiation such as acrylic.

The shield may be a sheet, grill or mesh. The shields may be structurally reinforced using aluminium or aluminium alloy supports.

Reflecting means (not shown) may be used within the horizontal tanning chamber to reflect any stray tanning radiation towards the user. The reflecting means may be positioned behind and/or to the side of the tanning lamps (4) arranged within the upper and lower portions (1,2) of the tanning chamber. The reflecting means are formed from a material with a surface that is reflective to tanning radiation, such as flexible polished sheet metal or metalised plastics.

The horizontal tanning chamber may include cooling means (not shown) to dissipate the heat generated by the tanning lamps (4) in order to ensure that the user does not become too hot when the chamber is in use. The cooling means may include cooling fins and/or a fan.

The horizontal tanning chamber includes control means to control the operation of the tanning lamps (4) on the upper and lower portions (1,2)). The control means include ballast or electronic chokes, capacitors, starters and timers. The control means are often accommodated within the base (6) and/or within the walls of the upper and lower tanning portions (1,2). Certain control means may be attached to the outer walls of the chamber so that they are easily accessible for the user. The tanning lamps (4) on the upper and lower portions (1,2) may be electrically connected to the control means via lamp holders (not shown).

The tanning unit (10) may be operated independently of or together with the horizontal tanning chamber. The control means of the tanning unit (10) may be arranged such that the tanning lamps (4) of the tanning unit (10) can be operated independently and separately to the tanning lamps (4) of the tanning chamber. Thus, the tanning unit (10) may be operated for a different period of time to the tanning chamber. Conversely, the control means of the tanning unit (10) may be arranged such that the tanning lamps (4) of the tanning unit (10) can be operated together with or by the chamber. Thus, the tanning unit (10) may be operated in conjunction with the tanning chamber.

The horizontal tanning unit may be powered by an external power supply or internally fitted battery (not shown). The tanning unit (10) may be powered separately from or together with the tanning chamber.

The tanning chamber of Figure 7 includes a vertical wall (7) and floor (8). At least one door (9) is formed within the wall (7) so that a user can enter and exit the chamber. Tanning lamps (4) are arranged on the inner surface of the wall (7) and door (9). A tanning unit (10) is arranged on the floor (8) of the chamber. The tanning unit (10) is located on the floor (8) so that the legs of the user can straddle the unit (10) when standing up within the vertical tanning chamber.

The tanning unit (10) may be retrofitted within the tanning chamber, so that it can be added or removed as required. Alternatively, the tanning unit (10) may be integrally fitted within the tanning chamber.

The internal configuration of the chamber is generally circular in shape. The vertical tanning chamber may stand between approximately 2100mm and approximately 2400mm tall. The internal configuration may have a diameter of approximately 820mm. The external configuration of the chamber may also be generally circular and have an overall diameter of approximately H50mm. Alternatively, the external configuration of the chamber may be rectangular or square. If the external configuration of the vertical tanning chamber is rectangular the width may be approximately 1200mm and the depth approximately 1150mm.

The tanning lamps (4) of the vertical tanning chamber emit UVA and UVB radiation. The tanning lamps (4) may be elongate tubes, such as low-pressure fluorescent tubes. The tanning chamber may include high output tubes that range from approximately 100W to approximately 200W and/or booster tubes that range from approximately 15W to approximately 36W. The vertical tanning chamber may include approximately 40 to approximately 190 tanning lamps (4). The lamps (4) may be aligned vertically, perpendicular to the place of the floor (8), and extend the full length of the person. Booster tubes may be added to the lower half and/or upper half of the chamber to boost the tanning of the face and/or the outer surfaces of the user's legs. Booster tubes may also be added to the middle section of the chamber to boost the tanning of the body.

The tanning lamps (4) may be held in place by lamp holders (not shown). The lamps (4) may be arranged in a uniform row or staggered configuration. Figure 7 shows the tanning lamps (2) of the vertical tanning chamber arranged in vertical uniform rows.

The tanning lamps (4) may be arranged at least a 'finger width' apart to facilitate installation and removal of the lamps (4).

As mentioned above, the internal configuration of the vertical tanning chamber is generally circular in shape. When the at least one door (9) is closed a void is formed that is essentially tubular in shape. When the vertical tanning chamber and tanning unit (10) are in use, a person stands on the floor (8) within the void and straddles the tanning unit (10). The tanning lamps (4) of the tanning chamber are arranged so that the user is generally surrounded by lamps (4) such that the entire length of the body is irradiated from almost every direction. The inside surface of each leg faces the outer surfaces (12) of the tanning unit (10) and the outside surface of each leg faces the tanning lamps (4) arranged on the inner surface of the wall (7) and door (9). Since radiation is able to reach all parts of the user's legs they are sun-tanned more evenly. When a user straddles the tanning unit (10) in a standing position the outside surface of each leg is located closer to the tanning lamps (4) on the wall and door than in a conventional vertical tanning chamber, Consequently, since the lower body of the user is located closer to the tanning lamps (4) of the vertical tanning chamber, the overall suntan across the entire body is more uniform.

Shields (not shown) may be mounted over the inner surface of the wall (7) and door (9) to prevent the user from having direct contact with the tanning lamps (4).

Reflecting means (not shown) may also be used within the vertical tanning chamber to reflect any stray tanning radiation towards the user. The reflecting means may be positioned behind and/or to the side of the tanning lamps (4) arranged on the inner surface of the wall (7) and door (9).

The vertical tanning chamber may include stabilising means (not shown). The stabilising means are shaped and arranged such that a user may hold onto them to help stabilise him/her while standing in the chamber. The stabilising means may be positioned above the user's head such that he/she has to lift his/her arms in order to hold them. As a result, the user's arm can be uniformly sun-tanned. When a user lifts his/her arms above his/her head to hold onto the stabilising means tanning radiation is able to reach both the inner and outer portions of the arms.

The vertical tanning chamber may include cooling means (not shown) to dissipate heat generated by the tanning lamps (4) in order to ensure the user does not become too hot when the chamber is in use. The cooling means may include cooling fins and/or a fan.

The vertical tanning chamber includes control means (not shown) to control the operation of the tanning lamps (4) arranged on the inner surface of the wall (7) and (8). The control means include ballast or electronic chokes, capacitors, starters and timers. The control means may be accommodated in the base (6), floor (8), door (9) and/or walls (7). Certain control means may be attached to the outer surfaces of the horizontal tanning chamber so that they are easily accessible by the user. The tanning lamps (4) on the wall (7) and door (8) may be electrically connected to the control means via lamp holders (not shown).

The tanning unit (10) may be operated independently of or together with the chamber. The control means of the tanning unit (10) may be arranged such that the tanning lamps (4) of the tanning unit (10) can be operated independently and separately to the tanning lamps (4) of the conventional tanning chamber. Thus, the tanning unit (10) may be operated for a different period of time to the tanning chamber. Conversely, the control means of the tanning unit (10) may be arranged such that the tanning lamps (4) of the tanning unit (10) can be operated together with or by the chamber. Thus, the tanning unit (10) may be operated in conjunction with the tanning chamber.

The vertical tanning chamber may be powered by an external power supply or by an internally fitted battery (not shown). The tanning unit (10) may be powered separately from or together with the tanning chamber.

Embodiments of the present invention suntan the inside surface of the legs of a person. Embodiments of the present invention provide a tanning unit for legs. When a person straddles the tanning unit for legs the inside surface of the person's legs face tanning radiation emitting means. Thus, in operation the tanning unit for legs irradiates the inside surface of the user's legs with tanning radiation.

Embodiments of the present invention uniformly suntan the inside and outside surfaces of the legs of a person. Embodiments of the present invention provide a tanning chamber that includes a tanning unit for legs. When a person stands or lies within a tanning chamber and straddles the tanning unit for legs both the inside and outside surfaces of the legs face tanning radiation emitting means. Thus, in operation the tanning chamber and tanning unit for legs irradiate both the inside and outside surfaces of the user's legs with tanning radiation.

Embodiments of the present invention uniformly suntan the upper and lower body of a person. Embodiments of the present invention provide a tanning chamber that includes a tanning unit for legs. When a person stands or lies within a tanning chamber and straddles the tanning unit for legs both the front, back and sides of the upper body face tanning radiation emitting means and both the inside and outside surfaces of the legs face tanning radiation emitting means. As a result of straddling the tanning unit for legs, the legs are arranged so that the lower body is approximately the same width as the upper body. The outside surface of the legs is located approximately the same distance from tanning radiation emitting means as the upper body. Thus, in operation the tanning chamber and tanning unit for legs irradiate generally the entire body with tanning radiation.

Although the above preferred embodiments have been described in detail with respect to the accompanying drawings it will be understood that the present invention is not limited to the details described herein. Variations and modifications may be made without departing from the scope of the present invention.

## Claims

1. A tanning unit for legs comprising a housing (11) with at least one outer surface (12) and at least one tanning lamp (4) wherein the at least one lamp (4) is arranged within the housing (11), and disposed such that, during operation, tanning radiation is emitted through the at least one outer surface (12), and wherein the housing (11) is shaped to enable a user to straddle the tanning unit such that the inside surface of each leg of the user is adjacent the at least one outer surface, whereby, in operation, the inside surfaces of both legs are simultaneously subject to said tunning radiation.

2. A tanning unit for legs as claimed in claim 1 wherein at least one tanning lamp (4) is arranged within the housing (11) and disposed adjacent the outer surface (12).

3. A tanning unit for legs as claimed in claims 1 or 2 wherein the housing (11) has at least two outer surfaces (12).

4. A tanning unit for legs as claimed in claim 3 wherein the at least one tanning lamp (4) is arranged within the housing (11) and disposed adjacent each one of the at least two outer surfaces (12).

5. A tanning unit for legs as claimed in any preceding claim further comprising control means to control the operation of the at least one tanning lamp (4).

6. A tanning unit for legs as claimed in any preceding claim further comprising at least two shields each mounted over one of the at least two outer surfaces and formed from material transmissive to tanning radiation.

7. A tanning chamber including a tanning unit for legs as defined in any of claims 1 to 6.

8. A tanning chamber as claimed in claim 6 wherein the tanning unit for legs is integrally fitted within the chamber.

9. A tanning chamber as claimed in claims 7 or 8 wherein the tanning unit for legs.is operable independently of the chamber.

10. A tanning chamber as claimed in claims 7 or 8 wherein the tanning unit for legs is operable together with the chamber.

11. A tanning chamber as claimed in any of claims 7 to 10 wherein the tanning chamber is a vertical tanning chamber to tan a user while in a standing position.

12. A tanning chamber as claimed in claim 11 further comprising a housing with a floor section wherein the tanning unit for legs is located on the floor section such that a user may straddle the unit while standing.

13. A tanning chamber as claimed in any of claims 7 to 10 wherein the tanning chamber is a horizontal tanning chamber to tan a user while in a lying position.

14. A tanning chamber as claimed in claim 13 further comprising an upper portion and a lower portion, wherein the tanning unit for legs is located at one end of the lower portion of the horizontal tanning chamber such that a user may straddle the unit while lying down.

15. A method of tanning legs using a tanning unit for legs as claimed in any of claims 1 to 6 comprising the steps of:
a user straddling the tanning unit such that the inside surface of each leg of the user is adjacent the at least one outer surface (12) of the tanning unit; and
irradiating the inside surface of each leg with tanning radiation when the tanning unit is in operation.

16. A method of tanning using a vertical tanning chamber including a tanning unit for legs as claimed in any of claims 11 or 12, when dependent on any of claims 1 to 10, comprising the steps of:
a user standing within the vertical tanning chamber and straddling the tanning unit for legs whereby the user is generally surrounded by tanning lamps (4) of the tanning chamber and the inside surface of each leg of the user is adjacent the at least one outer surface (12); and
irradiating, via the tanning lamps (4) of the vertical tanning chamber, the entire length of the user's body from a plurality of directions and irradiating, via the tanning unit for legs, the inside surface of each leg.

17. A method of tanning using a horizontal tanning chamber including a tanning unit
for legs as claimed in claims 13 or 14, when dependent on any of claims 1 to 10, comprising the steps of:
a user lying within the horizontal tanning chamber and straddling the tanning unit for legs whereby the user is generally surrounded by tanning lamps of the tanning chamber and the inside surface of each leg of the user is adjacent the at least one outer surface of the tanning unit; and
irradiating, via the tanning lamps (4) of the horizontal tanning, the entire length of the user's body from a plurality of directions and irradiating, via the tanning unit for legs, the inside surface of each leg.

## Patentansprüche

1. Eine Bräunungseinheit für Beine, die ein Gehäuse (11) mit mindestens einer äußeren Oberfläche (12) und mindestens einer Bräunungslampe (4) umfasst, wobei die mindestens eine Lampe (4) im Gehäuse (11) angeordnet und so eingerichtet ist, dass während des Betriebs Bräunungsstrahlung durch die mindestens eine äußere Oberfläche (12) ausgesendet wird, und wobei das Gehäuse (11) so gebildet ist, dass ein Benutzer in der Lage ist, sich mit gespreizten Beinen so über der Bräunungseinheit zu positionieren, dass die innere Oberfläche jedes Beins des Benutzers neben der mindestens einen äußeren Oberfläche liegt, wodurch während des Betriebs die inneren Oberflächen beider Beine gleichzeitig der genannten Bräunungsstrahlung ausgesetzt werden können.

2. Eine Bräunungseinheit für Beine, wie in Anspruch 1 beansprucht, wobei mindestens eine Bräunungslampe (4) im Gehäuse (11) angeordnet und neben der äußeren Oberfläche (12) eingerichtet ist.

3. Eine Bräunungseinheit für Beine, wie in den Ansprüchen 1 oder 2 beansprucht, wobei das Gehäuse (11) mindestens zwei äußere Oberflächen (12) aufweist.

4. Eine Bräunungseinheit für Beine, wie in Anspruch 3 beansprucht, wobei die mindestens eine Bräunungslampe (4) im Gehäuse (11) angeordnet und neben jeder der mindestens zwei äußeren Oberflächen (12) eingerichtet ist.

5. Eine Bräunungseinheit für Beine, wie in einem der vorhergehenden Ansprüche beansprucht, die ferner Steuermittel zur Steuerung des Betriebs der mindestens einen Bräunungslampe (4) umfasst.

6. Eine Bräunungseinheit für Beine, wie in einem der vorhergehenden Ansprüche beansprucht, die ferner mindestens zwei Schutzplatten umfasst, welche jeweils über einer der mindestens zwei äußeren Oberflächen montiert und aus Material geformt sind, das für Bräunungsstrahlung durchlässig ist.

7. Eine Bräunungskammer, die eine wie in einem der Ansprüche 1 bis 6 definierte Bräunungseinheit für Beine beinhaltet.

8. Eine Bräunungskammer, wie in Anspruch 6 beansprucht, wobei die Bräunungseinheit für Beine in die Kammer integriert eingebaut ist.

9. Eine Bräunungskammer, wie in den Ansprüchen 7 oder 8 beansprucht, wobei die Bräunungseinheit für Beine unabhängig von der Kammer bedient werden kann.

10. Eine Bräunungskammer, wie in den Ansprüchen 7 oder 8 beansprucht, wobei die Bräunungseinheit für Beine zusammen mit der Kammer bedient werden kann.

11. Eine Bräunungskammer, wie in den Ansprüchen 7 bis 10 beansprucht, wobei die Bräunungskammer eine senkrechte Bräunungskammer ist, die einen Benutzer bräunt, der sich in einer stehenden Position befindet.

12. Eine Bräunungskammer, wie in Anspruch 11 beansprucht, die ferner ein Gehäuse mit einem Bodenabschnitt umfasst, wobei sich die Bräunungseinheit für Beine auf dem Bodenabschnitt befindet, so dass ein Benutzer mit gespreizten Beinen über der Einheit stehen kann.

13. Eine Bräunungskammer, wie in den Ansprüchen 7 bis 10 beansprucht, wobei die Bräunungskammer eine waagerechte Bräunungskammer ist, die einen Benutzer bräunt, der sich in einer liegenden Position befindet.

14. Eine Bräunungskammer, wie in Anspruch 13 beansprucht, die ferner einen oberen Teil und einen unteren Teil umfasst, wobei sich die Bräunungseinheit für Beine an einem Ende des unteren Teils der waagerechten Kammer befindet, so dass der Benutzer mit gespreizten Beinen über der Einheit liegen kann.

15. Eine Methode zum Bräunen von Beinen unter Verwendung einer Bräunungseinheit für Beine, wie in einem der Ansprüche 1 bis 6 beansprucht, die die folgenden Schritte umfasst:
Ein Benutzer positioniert sich mit gespreizten Beinen auf/über der Bräunungseinheit, so dass die innere Oberfläche jedes Beins des Benutzers neben der mindestens einen äußeren Oberfläche (12) der Bräunungseinheit liegt; und
die innere Oberfläche jedes Beins wird mit Bräunungsstrahlung bestrahlt, wenn sich die Bräunungseinheit in Betrieb befindet.

16. Eine Bräunungsmethode unter Verwendung einer senkrechten Bräunungskammer, die eine Bräunungseinheit für Beine wie in den Ansprüchen 11 oder 12 beansprucht beinhaltet, welche, wenn von den Ansprüchen 1 bis 10 abhängig, die folgenden Schritte umfasst:
Ein Benutzer steht mit gespreizten Beinen in der senkrechten Bräunungskammer über der Bräunungseinheit für Beine, wobei der Benutzer generell von den Bräunungslampen (4) der Bräunungskammer umgeben ist und die innere Oberfläche jedes Beins des Benutzers neben der mindestens einen äußeren Oberfläche (12) liegt; und
die gesamte Körperlänge des Benutzers wird über die Bräunungslampen (4) der senkrechten Bräunungskammer aus einer Vielzahl von Richtungen bestrahlt und die innere Oberfläche jedes Beins wird über die Bräunungseinheit für Beine bestrahlt.

17. Eine Bräunungsmethode unter Verwendung einer waagerechten Bräunungskammer, die eine Bräunungseinheit für Beine wie in den Ansprüchen 13 oder 14 beansprucht beinhaltet, welche, wenn von den Ansprüchen 1 bis 10 abhängig, die folgenden Schritte umfasst:
Ein Benutzer liegt in der waagerechten Bräunungskammer und spreizt seine Beine über der Bräunungseinheit für Beine wobei der Benutzer generell von Bräunungslampen der Bräunungskammer umgeben ist und die innere Oberfläche jedes Beins des Benutzers neben der mindestens einen äußeren Oberfläche der Bräunungseinheit liegt; und
die gesamte Körperlänge des Benutzers wird über die Bräunungslampen (4) der waagerechten Bräunungseinheit aus einer Vielzahl von Richtungen bestrahlt und die innere Oberfläche jedes Beins wird über die Bräunungseinheit für Beine bestrahlt.

## Revendications

1. Unité de bronzage pour jambes comprenant un carter (11) ayant au moins une surface externe (12) et au moins une lampe de bronzage (4), dans laquelle la au moins une lampe (4) est agencée à l'intérieur du carter (11) et est disposée de sorte que, pendant le fonctionnement, le rayonnement de bronzage soit émis à travers la au moins une surface externe (12), et dans laquelle le carter (11) est façonné de manière à permettre à un utilisateur de chevaucher l'unité de bronzage pour que la surface interne de chaque jambe de l'utilisateur soit adjacente à la au moins une surface externe, cas dans lequel, en cours de fonctionnement, les surfaces internes des deux jambes soient simultanément soumises audit rayonnement de bronzage.

2. Unité de bronzage pour jambes, selon la revendication 1, dans laquelle au moins une lampe de bronzage (4) est agencée à l'intérieur du carter (11) et est disposée en position adjacente à la surface externe (12).

3. Unité de bronzage pour jambes, selon les revendications 1 ou 2, dans laquelle le carter (11) possède au moins deux surfaces externes (12).

4. Unité de bronzage pour jambes, selon la revendication 3, dans laquelle la au moins une lampe de bronzage (4) est agencée à l'intérieur du carter (11) et est disposée en position adjacente à chacune des au moins deux surfaces externes (12).

5. Unité de bronzage pour jambes, selon l'une quelconque des revendications précédentes, comprenant en outre un moyen de commande afin de commander le fonctionnement de la au moins une lampe de bronzage (4).

6. Unité de bronzage pour jambes, selon l'une quelconque des revendications précédentes, comprenant en outre deux écrans de protection, chacun étant monté au-dessus de l'une des au moins deux surfaces externes et fabriqués dans un matériau capable de laisser passer le rayonnement de bronzage.

7. Chambre de bronzage comprenant une unité de bronzage pour jambes, telle qu'elle est définie dans l'une quelconque des revendications 1 à 6.

8. Chambre de bronzage, selon la revendication 6, dans laquelle l'unité de bronzage pour jambes est montée de façon solidaire à l'intérieur de la chambre.

9. Chambre de bronzage, selon les revendications 7 ou 8, dans laquelle l'unité de bronzage pour jambes est capable de fonctionner indépendamment de la chambre.

10. Chambre de bronzage, selon les revendications 7 ou 8, dans laquelle l'unité de bronzage pour jambes est capable de fonctionner en conjonction avec la chambre.

11. Chambre de bronzage, selon l'une quelconque des revendications 7 à 10, dans laquelle la chambre de bronzage est une chambre de bronzage verticale permettant de faire bronzer un utilisateur pendant qu'il est en position debout.

12. Chambre de bronzage, selon la revendication 11, comprenant en outre un carter ayant une section plancher dans laquelle l'unité de bronzage pour jambes est positionnée sur la section plancher de telle sorte qu'un utilisateur puisse chevaucher l'unité pendant qu'il se tient debout.

13. Chambre de bronzage, selon l'une quelconque des revendications 7 à 10, dans laquelle la chambre de bronzage est une chambre de bronzage horizontale permettant de faire bronzer un utilisateur pendant qu'il est en position couchée.

14. Chambre de bronzage, selon la revendication 13, comprenant en outre une section supérieure et une section inférieure, dans laquelle l'unité de bronzage pour jambes est positionnée à l'une des extrémités de la section inférieure de la chambre de bronzage horizontale de telle sorte que l'utilisateur puisse chevaucher l'unité pendant qu'il est en position couchée.

15. Procédé de bronzage de jambes faisant intervenir une unité de bronzage pour jambes, selon l'une quelconque des revendications 1 à 6 comprenant les étapes suivantes :
un utilisateur qui chevauche l'unité de bronzage de telle sorte que la surface interne de chaque jambe de l'utilisateur soit adjacente à la au moins une surface externe (12) de l'unité de bronzage ; et
l'irradiation de la surface interne de chaque jambe à l'aide d'un rayonnement de bronzage lorsque l'unité de bronzage est en fonctionnement.

16. Procédé de bronzage faisant intervenir une chambre de bronzage verticale comportant une unité de bronzage pour jambes, selon l'une quelconque des revendications 11 ou 12, quand elle dépend de l'une quelconque des revendications 1 à 10 comprenant les étapes suivantes :
un utilisateur qui se tient debout à l'intérieur de la chambre de bronzage verticale et qui chevauche l'unité de bronzage pour jambes, cas dans lequel l'utilisateur est généralement entouré de lampes de bronzage (4) de la chambre de bronzage et la surface interne de chaque jambe de l'utilisateur est adjacente à la au moins une surface externe (12) ; et
l'irradiation, par l'intermédiaire des lampes de bronzage (4) de la chambre de bronzage verticale, de la longueur tout entière du corps de l'utilisateur à partir d'une pluralité de directions, et l'irradiation, par l'intermédiaire de l'unité de bronzage pour jambes, de la surface interne de chaque jambe.

17. Procédé de bronzage faisant intervenir une chambre de bronzage horizontale comportant une unité de bronzage pour jambes, selon les revendications 13 ou 14, quand elle dépend de l'une quelconque des revendications 1 à 10 comprenant les étapes suivantes :
un utilisateur qui est en position couchée à l'intérieur de la chambre de bronzage horizontale et qui chevauche l'unité de bronzage pour jambes, cas dans lequel l'utilisateur est généralement entouré de lampes de bronzage de la chambre de bronzage et la surface interne de chaque jambe de l'utilisateur est adjacente à la au moins une surface externe de l'unité de bronzage ; et
l'irradiation, par l'intermédiaire des lampes de bronzage (4) de la chambre de bronzage horizontale, de la longueur tout entière du corps de l'utilisateur à partir d'une pluralité de directions, et l'irradiation, par l'intermédiaire de l'unité de bronzage pour jambes, de la surface interne de chaque jambe.
